# EUROPEAN PATENT SPECIFICATION

(11) **EP 3 682 940 B1**
(45) Date of publication and mention of the grant of the patent: **30.08.2023**
(21) Application number: 19207487.0
(22) Date of filing: 06.11.2019
(51) Int. Cl.: A61M 39/24, A61F 5/44, A61F 5/441

(54) **BACKFLOW PREVENTION DEVICE**
RÜCKFLUSSVERHINDERUNGSVORRICHTUNG
DISPOSITIF DE PRÉVENTION DE REFLUX

(30) Priority: 18.01.2019 KR 20190006531
(43) Date of publication of application: 22.07.2020
(73) Proprietor: Curaco, Inc., Gyeonggi-do, 13210 (KR)
(72) Inventor: LEE, Hoonsang, 12787 GYEONGGI-DO (KR); LEE, Hosang, 06218 SEOUL (KR); CHOI, Sungpil, 07257 Seoul (KR); PARK, Minjae, 15861 Gyeonggi-do (KR)
(74) Representative: Ter Meer Steinmeister & Partner

(56) References cited:
- JP-A- H0 884 688
- KR-U- 20100 003 849
- US-A1- 2013 036 544

## Description

This application claims priority of KR No. 10-2019-0006531, filed on 01.18,2019.

### BACKGROUND OF THE INVENTION

The present invention relates to a backflow prevention device. More particularly, the invention relates to a device which is connected to a dreg disposal device to effectively store only dreg.

It is very difficult for the old and weak or a patient himself or herself to dispose of urine and feces due to physical or mental discomfort, so that the assistance of a guardian or a caregiver is needed to dispose of excretion. However, such a process of disposing of the excretion causes a lot of stress and inconvenience to the old and weak or the patient as well as the guardian or the caregiver.

In order to solve this problem, various excretion disposal devices are being developed. Recently, the excretion disposal device is not limited to dispose of the excretion, has various additional functions such as a bidet function or a drying function, and a restriction on space for storing the excretion for the reason of a user's wearing sensation or the like causes a problem.

Furthermore, the dregs stored in the excretion disposal device are inevitably disposed of in a space adjacent to the old and weak or the patient, so that it is not easy to completely eliminate psychological burdens.

The present invention allows only dregs to be effectively stored and simultaneously makes it easy to dispose of the dregs, thus solving the above-described problems.
[Cited Document] Korean Patent Publication No. 10-0588297

KR 2010 0003849 U relates to a mattress having an automatic defection treatment apparatus with a waste collection can. The waste collection can has a housing, an inlet to allow the excreta to flow into the housing and an outlet to suck air out of the waste collection can, in order that the excretion flows smoothly from a toilet seat through the inlet into the waste collection can. The inlet and the outlet are arranged at a cover of the housing. Inside the housing is a separating diaphragm protruding downward from the lower surface of the cover.

JP H08 84688 relates to a urine excretion bag having a bag body with a slit-like opening part, which can be opened and closed by means of a zipper. Inside the bag body a front and a back cloth piece is fixed to a linear fixing wire, placed next to the opening part. The front and back cloth pieces are forming a middle bag that prevents the excretion to flow back to the opening part, especially in case the excretion bag is pressurized by an external force.

US 2013/036544 A1 discloses an apparatus for automatically treating excrement, capable of receiving excrement of a patient and automatically treating the excrement, including a vacuum pump for facilitating the flow of excrements and the discharge of air.

### SUMMARY OF THE INVENTION

The present invention has been made in an effort to solve the above-described problems associated with prior art. An object of the present invention is to provide a backflow prevention device, which is capable of storing dregs and easily disposing of the dreg

Another object of the present invention is to provide a backflow prevention device, which is operated in conjunction with an excretion disposal device to effectively store only the excretion. The objects of the present invention are not limited to the above-mentioned objects, and other objects which are not mentioned above will be clearly understood by those skilled in the art from the following description.

In an aspect, the present invention is intended to provide a backflow prevention device including a reservoir defining a space to store dregs therein, an inflow unit having an inlet hole formed therein and defining a path to allow the dregs to move through the inlet hole into the reservoir, a discharge unit having an outlet hole formed therein and defining a path to allow air in the reservoir to be discharged through the outlet hole, and a barrier unit provided in the reservoir to change a flow direction of at least either of the dregs and the air at least once and thereby prevent the dregs from flowing into the discharge unit.

The barrier unit includes a first barrier part formed from an outside of the inflow unit towards an inside of the reservoir.

The first barrier part may be inclined in a dreg falling direction.

The barrier unit includes a second barrier part formed from the inside of the reservoir towards the outside of the inflow unit.

The barrier unit includes a first barrier part formed from the outside of the inflow unit towards the inside of the reservoir, a second barrier part formed from the inside of the reservoir towards the outside of the inflow unit, and a third barrier part formed from the outside of the inflow unit towards the inside of the reservoir, wherein the second barrier part may be provided between the first barrier part and the third barrier part.

At least any one of the first barrier part, the second barrier part and the third barrier part may be inclined in the dreg falling direction.

The backflow prevention device may further include a cover unit detachably coupled to the reservoir, with a handle member rotatably provided on the cover unit, wherein the inflow unit and the discharge unit may be formed to pass through a part of the cover unit.

The inflow unit may be moved in a direction opposite to the dreg falling direction by rotation of the handle member, and may block a space between the first barrier part and the second barrier part.

When the handle member may be rotated and thus an external force may act in a direction opposite to the dreg falling direction, the inflow unit may move in a direction where the external force acts, thus blocking the space between the first barrier part and the second barrier part.

The inflow unit is configured such that a first end thereof is movable, and, when an external force acts on the inflow unit in a direction opposite to the dreg falling direction, the inflow unit may move in a direction where the external force acts, thus blocking the space between the first barrier part and the second barrier part.

The discharge unit may be coupled to a power unit to suck air from an inside of the reservoir.

The discharge unit may include a filter member to filter the air.

### BRIEF DESCRIPTION OF THE DRAWINGS

FIG. 1 is a perspective view illustrating a backflow prevention device;
FIG. 2 is a sectional view illustrating the backflow prevention device;
FIGS. 3A and 3B and FIGS. 4A and 4B are views illustrating a first embodiment of a cover unit;
FIGS. 5A and 5B and FIGS. 6A and 6B are views illustrating a second embodiment of a cover unit; and
FIGS. 7A and 7B and FIGS. 8A and 8B are views illustrating a third embodiment of a cover unit in accordance with the present invention.

### DETAILED DESCRIPTION OF THE EMBODIMENTS

Hereinafter, preferred embodiments of the present invention will be described in detail with reference to the accompanying drawings. In describing the embodiments, the same reference numerals denote the same components throughout the drawings.

Further, in the description of embodiments of the present invention, the same names and the same reference numerals are used for components having the same function, which are not substantially equal to components of the related art.

Furthermore, terms used in the embodiments of the present invention are employed to merely describe specific embodiments, but are not intended to limit the present invention. Singular expression includes plural expression unless otherwise specified clearly.

Moreover, in the embodiment of the present invention, it should be understood that terms such as "comprise" or "have" are intended to indicate that there are features, numbers, steps, operations, components, parts or combinations thereof described in the specification, and the possibility of the presence or addition of one or more other features, numbers, steps, operations, components, parts or combinations thereof is not excluded.

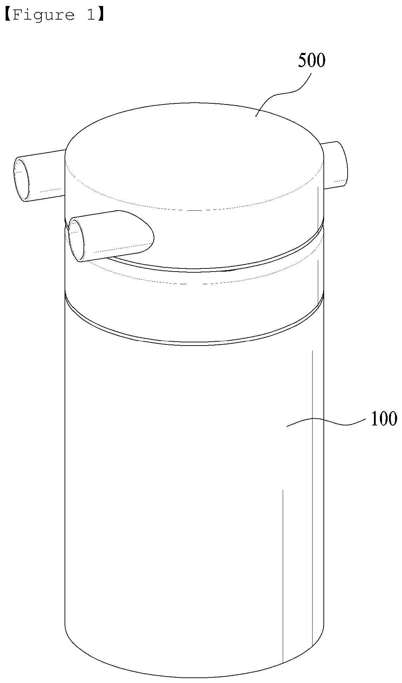

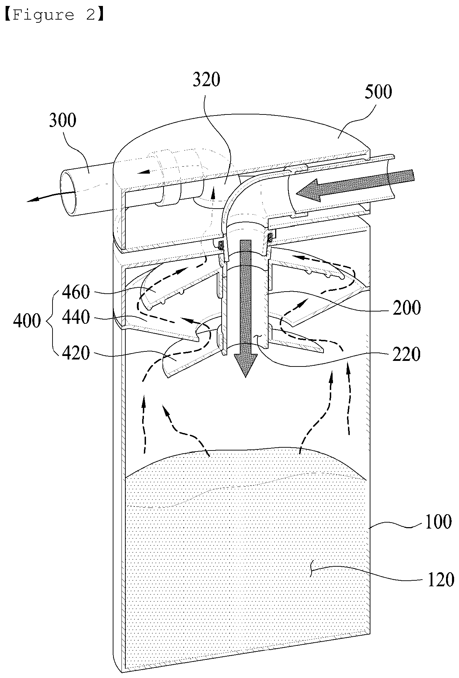

A backflow prevention device will be described with reference to FIGS. 1 and 2. FIG. 1 is a perspective view illustrating the backflow prevention device in accordance with the present invention, and FIG. 2 is a sectional view illustrating the backflow prevention device in accordance with the present invention.

Referring to FIGS. 1 and 2, the backflow prevention device may include a reservoir 100, an inflow unit 200, a discharge unit 300, and a barrier unit 400.

The reservoir 100 may define a storing space 120 that stores dregs therein. The reservoir 100 may be formed in various shapes, such as a cylindrical shape or a hexahedral shape, but is not limited to the shape of drawings.

The inflow unit 200 may have an inlet hole 220, and may provide a path to allow the dregs to move through the inlet hole 220 to the interior of the reservoir 100, namely, the storing space 120. For example, the inflow unit 200 may be connected to the excretion disposal device, so that dregs such as the excretion of the old and weak or a patient discharged through the excretion disposal device may be introduced into the storing space 120.

The discharge unit 300 may have an outlet hole 320, and may provide a path to allow air contained in the reservoir 100 to be discharged through the outlet hole 320. By discharging the air in the reservoir 100 through the discharge unit 300, the dregs may be smoothly moved through the inflow unit 200.

Preferably, the discharge unit 300 is coupled to a power unit to suck the air from the reservoir 100. Since the power unit is composed of a motor or the like, the air in the reservoir 100 can flow smoothly. In other words, the power unit can suck the air from the reservoir 100 to allow the dregs to be introduced through the inflow unit 200. However, the power unit is not limited to a specific number, performance and the like as long as the power unit performs the above-described function.

The barrier unit 400 may be provided in the reservoir 100, and may change the flow direction of at least either of the dregs and the air at least once to prevent the dregs from being introduced into the discharge unit 300.

The barrier unit 400 acts like a labyrinth in the flow of the dregs and the air. Here, when the flow direction of the dregs is changed by the barrier unit 400, the dregs do not approach the discharge unit 300 by weight. However, the air of the storing space 120 may be discharged through the discharge unit 300 by flowability even when the flow direction is changed by the barrier unit 400. That is, only the air among the dregs and the air stored in the reservoir 100 may be selectively discharged through the discharge unit 300 by the barrier unit 400.

The barrier unit 400 may be formed in various shapes without being limited to a certain shape.

Here, the discharge unit 300 may include a filter member.

The filter member may be provided in the discharge unit 300 to filter the air. The air in the reservoir 100 is likely to have an unpleasant odor by the dregs, and the filter member may filter the odor or particles of the air to prevent damage caused by the odor. The location, type and others of the filter member may vary depending on the use environment.

According to the first embodiment, the barrier unit 400 may include a first barrier part 420.

The first barrier part 420 may be formed from the outside of the inflow unit 200 towards the inside of the reservoir 100. Preferably, the first barrier part 420 is formed to be inclined in a direction where the dregs fall.

Since the first barrier part 420 is formed from the outside of the inflow unit 200 towards the inside of the reservoir 100, the flow direction of at least either of the dregs and the air is changed at least once.

The above-mentioned configuration may serve as a primary barrier even when the dregs discharged from one end of the inflow unit 200 spatter due to interaction with the dregs that are already stored in the storing space 120. Since the dregs are discharged from one end of the inflow unit 200, the dregs are most likely to spatter at a position close to one end of the inflow unit 200. Furthermore, since the first barrier part 420 is formed to be inclined in a direction where the dregs fall, such a function can be more effectively performed.

According to the second embodiment, the barrier unit 400 may include a second barrier part 440.

The second barrier part 440 may be formed from the inside of the reservoir 100 towards the outside of the inflow unit 200. Preferably, the second barrier part 440 is formed to be inclined in a direction where the dregs fall. The second barrier part 440 may be different only in position from the first barrier part 420. The function of the second barrier part 440 for changing the flow direction of at least either of the dregs and the air at least once and thereby preventing the dregs from flowing into the discharge unit 300 may remain the same as the function of the first barrier part 420.

According to the third embodiment of the present invention, the barrier unit 400 may include the first barrier part 420, the second barrier part 440, and a third barrier part 460.

The first barrier part 420 may be formed from the outside of the inflow unit 200 towards the inside of the reservoir 100. The second barrier part 440 may be formed from the inside of the reservoir 100 towards the outside of the inflow unit 200. The third barrier part 460 may be formed from the outside of the inflow unit 200 towards the inside of the reservoir 100. Furthermore, the second barrier part 440 is preferably provided between the first barrier part 420 and the third barrier part 460.

Since the number of times in which the flow direction of at least either of the dregs and the air is changed by the first barrier part 420, the second barrier part 440, and the third barrier part 460 is increased, the function of the barrier unit 400 may be further reinforced. To be more specific, the flow direction of at least either of the dregs and the air may be changed at a lower portion of the first barrier part 420, an upper portion of the first barrier part 420, an upper portion of the second barrier part 440 and an upper portion of the third barrier part 460. Therefore, even if the flow direction is changed, the dregs do not approach the discharge unit 300 by weight. However, when the flow direction is changed by the barrier unit 400, the air of the storing space 120 may be discharged through the discharge unit 300 by the flowability.

Furthermore, at least one of the first barrier part 420, the second barrier part 440 and the third barrier part 460 is preferably inclined in a direction where the dregs fall. The reason why at least one of the first barrier part 420, the second barrier part 440 and the third barrier part 460 is formed to be inclined in the direction where the dregs fall is because the above-described function can be more effectively performed.

The foregoing description lists some of several embodiments. The barrier unit 400 may be composed of only the first barrier part 420 and the second barrier part 440. By the first barrier part 420 and the second barrier part 440, the same function and effect can be realized.

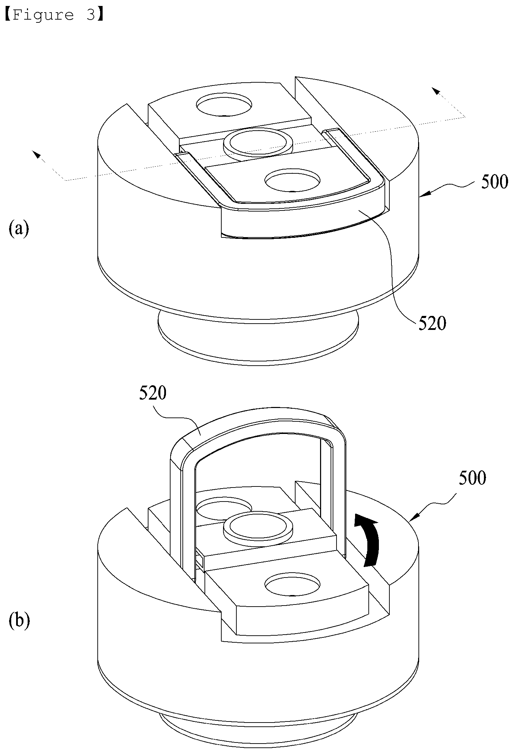

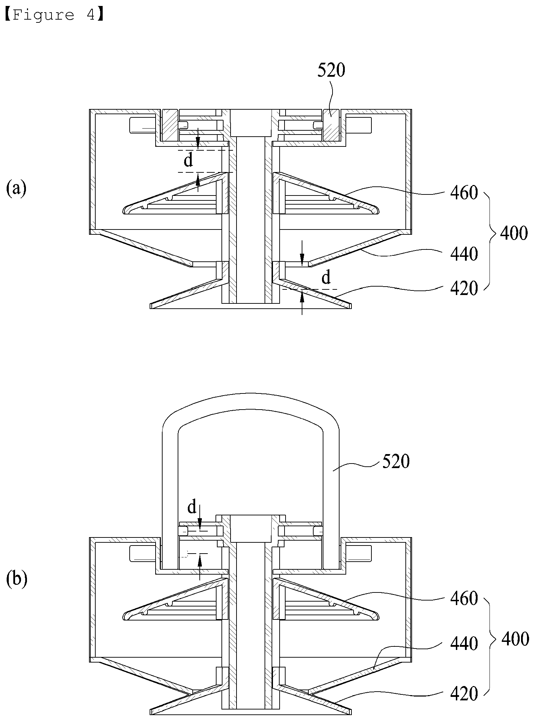

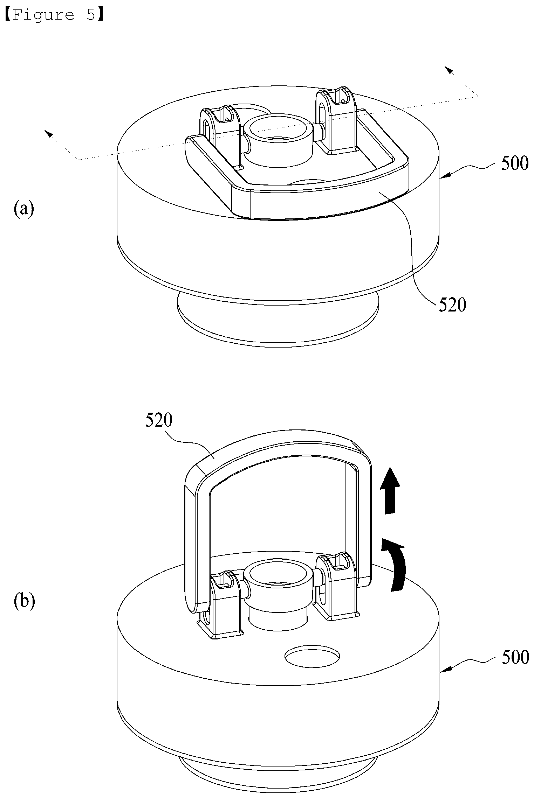

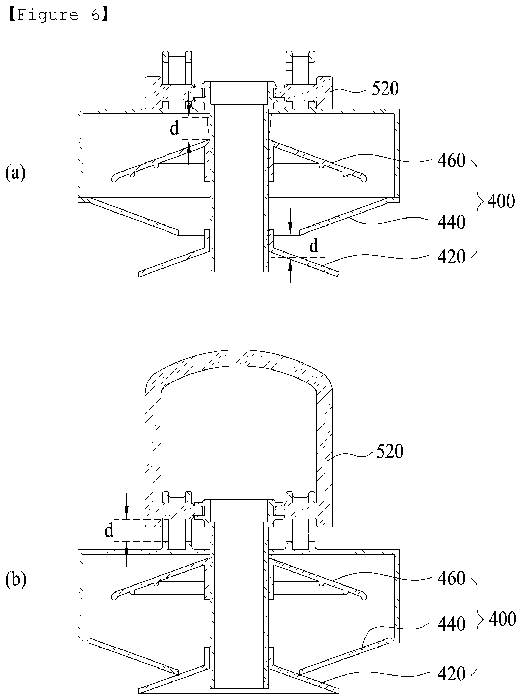

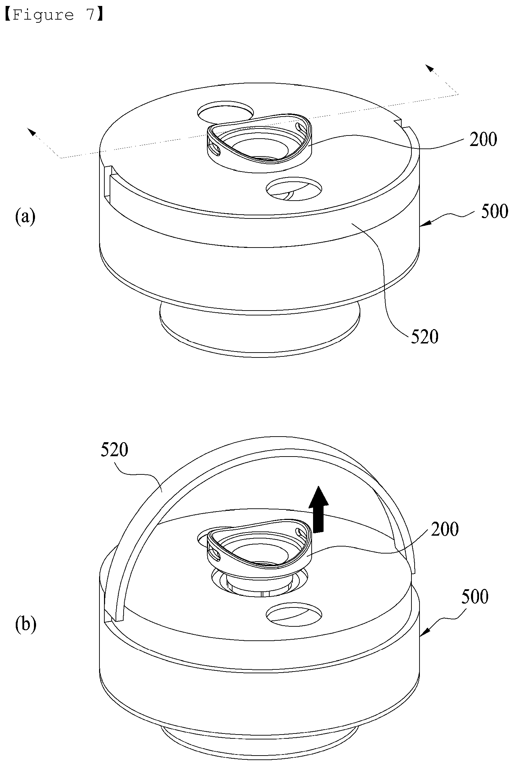

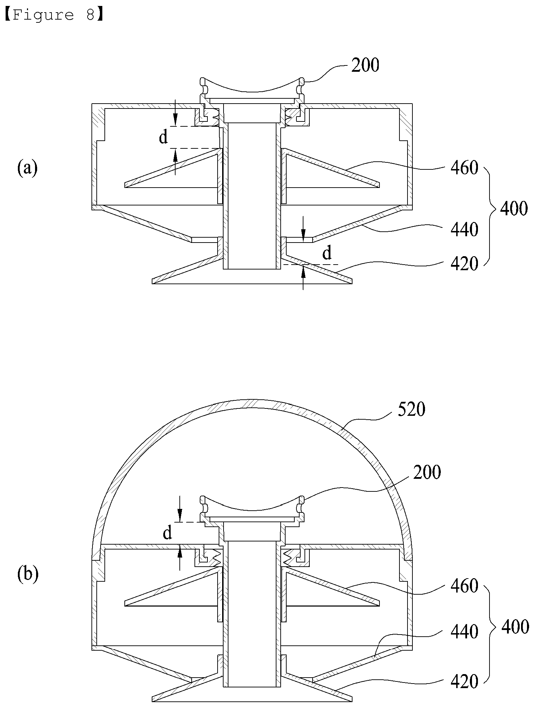

The configuration and operation of the cover unit according to the present invention will be described with reference to FIGS. 3A and 3B to FIGS. 8A and 8B. FIGS. 3A and 3B and FIGS. 4A and 4B are views illustrating a first embodiment of the cover unit, and FIGS. 5A and 5B and FIGS. 6A and 6B are views illustrating a second embodiment of the cover unit, FIGS. 7A and 7B and FIGS. 8A and 8B are views illustrating a third embodiment of the cover unit in accordance with the present invention.

The backflow prevention device of the present invention may include the cover unit 500.

The cover unit 500 may be detachably coupled to the reservoir 100, and a handle member 520 may be rotatably formed thereon. The inflow unit 200 and the discharge unit 300 may be formed to pass through a part of the cover unit 500. A user may separate the cover unit 500 from the reservoir 100 to easily dispose of the dregs stored in the reservoir 100. Furthermore, the cover unit 500 may be coupled to the reservoir 100, so that the backflow prevention device of the present invention may be used.

The shape where the inflow unit 200 and the discharge unit 300 pass through a part of the cover unit 500 may be freely changed for the ease of cleaning, the convenience of coupling and the like, without being limited to the drawings.

According to the first embodiment of the cover unit 500 shown in FIGS. 3A and 3B and FIGS. 4A and 4B, the inflow unit 200 may move in a direction opposite to the dreg falling direction in conjunction with the rotation of the handle member 520, and may block a space between the first barrier part 420 and the second barrier part 440.

To be more specific, the handle member 520 may be formed so that one end thereof is rotatable by external force. Further, a part of the other end of the handle member 520 may protrude to be inserted into the cover unit 500. The protruding part of the handle member 520 is formed to be biased to one end by a distance 'd' from the other end. When one end of the handle member 520 rotates, the protruding part may rotate while drawing an arc having a radius 'd' about the other end of the handle member 520. Therefore, when one end of the handle member 520 is located to be perpendicular to the cover unit 500, a part into which the protruding part of the other end of the handle member 520 is inserted may rise by 'd'. Here, the protruding part of the other end of the handle member 520 is connected to the inflow unit 200, so that the inflow unit 200 may rise by 'd'. Furthermore, the first barrier part 420 and the second barrier part 440 are formed to be spaced apart from each other in consideration of the distance 'd', thus blocking the space between the first barrier part 420 and the second barrier part 440 by the rise of the inflow unit 200. In addition to the handle member shown in the drawings, two handle members 520 may be provided to be used with both hands.

If the third barrier part 460 is spaced apart from the cover unit 500 by 'd', the space between the third barrier part 460 and the inside of the cover unit 500 is blocked, thus performing a multiple sealing function. That is, the third barrier part 460 may also move in a direction opposite to the dreg falling direction, so that the space defined by the third barrier part 460 and the interior of the cover unit 500 may be blocked.

Referring to FIGS. 5A and 5B and FIGS. 6A and 6B, when the handle member 520 is rotated, so that external force acts in the direction opposite to the dreg falling direction, the inflow unit 200 may move in the direction where the external force acts, thus blocking the space between the first barrier part 420 and the second barrier part 440.

To be more specific, the handle member 520 may be formed so that one end thereof is rotatable by external force. Further, a part of the other end of the handle member 520 may protrude to be inserted into the cover unit 500. The other end of the handle member 520 may be formed to be movable by distance 'd' while one end of the handle member 520 being perpendicular to the cover unit 500. Here, since the protruding part of the other end of the handle member 520 is connected to the inflow unit 200, the inflow unit 200 may also move up by the same distance 'd' when the handle member 520 moves up by 'd'. Moreover, since the first barrier part 420 and the second barrier part 440 are spaced apart from each other in consideration of the distance 'd', the space between the first barrier part 420 and the second barrier part 440 may be blocked as the inflow unit 200 moves up.

Furthermore, if the third barrier part 460 is spaced apart from the cover unit 500 by the distance 'd', the space between the third barrier part 460 and the inside of the cover unit 500 may be blocked, thus performing a multiple sealing function. That is, the third barrier part 460 may also move in a direction opposite to the dreg falling direction, so that the space defined by the third barrier part 460 and the interior of the cover unit 500 may also be blocked.

Referring to FIGS. 7A and 7B and FIGS. 8A and 8B, according to the third embodiment of the cover unit 500 of the present invention, the inflow unit 200 is provided such that one end thereof is movable and, when external force acts on the inflow unit 200 in the direction opposite to the dreg falling direction, the inflow unit may move in the direction where the external force acts, thus blocking the space between the first barrier part 420 and the second barrier part 440.

To be more specific, a part of the first barrier part 420 and a part of the second barrier part 440 may be spaced apart from each other by a linear distance 'd'. If the external force acts on the inflow unit 200 in the direction opposite to the dreg falling direction, the inflow unit 200 may be moved by 'd' to seal the space between the first barrier part 420 and the second barrier part 440. Furthermore, a space between the third barrier part 460 and the outside of the inflow unit 200 may also be sealed in the same principle.

Moreover, the dregs can be discharged with the cover unit 500 being coupled to the reservoir 100, without the necessity of disassembling the cover unit 500.

Although the preferred embodiments of the present invention have been disclosed for illustrative purposes, those skilled in the art will appreciate that various modifications, additions and substitutions are possible, without departing from the scope of the invention as disclosed in the accompanying claims.

In order to solve the problems of the related art, the backflow prevention device of the present invention has the following effects.

First, it is possible to safely store only the introduced dregs and to prevent the dregs from being discharged.

Second, when the backflow prevention device is combined with the excretion disposal device, the moving path of excretion can be controlled, so that a guardian or a caregiver does not have to dispose of the excretion in a space adjacent to the old and weak or a patient, thus reducing psychological burdens between both sides.

Third, it is possible to easily dispose of the stored dregs without giving a person an unpleasant feeling.

The effects of the present invention are not limited to the above-mentioned effects, and other effects which are not mentioned above will be clearly understood by those skilled in the art from the claims.

## Claims

1. A backflow prevention device comprising:
a reservoir (100) defining a space to store dregs therein;
an inflow unit (200) having an inlet hole (220) formed therein, and defining a path to allow the dregs to move through the inlet hole (220) into the reservoir (100);
a discharge unit (300) having an outlet hole (320) formed therein, and defining a path to allow air in the reservoir (100) to be discharged through the outlet hole (320); and
a barrier unit (400) provided in the reservoir (100) to change a flow direction of at least either of the dregs and the air at least once and thereby prevent the dregs from flowing into the discharge unit (300)
**characterized in that** the barrier unit (400) comprises:
a first barrier part (420) formed from the outside of the inflow unit (200) towards the inside of the reservoir (100);
a second barrier part (440) formed from the inside of the reservoir (100) towards the outside of the inflow unit (200); and
a third barrier part (460) formed from the outside of the inflow unit (200) towards the inside of the reservoir (100),
wherein the second barrier part (440) is provided between the first barrier part (420) and the third barrier part (460),
, wherein the inflow unit (200) is provided such that a first end thereof is movable, and, when an external force acts on the inflow unit (200) in a direction opposite to the dreg falling direction, the inflow unit (200) moves in a direction where the external force acts, thus blocking the space between the first barrier part (420) and the second barrier part (440).

2. The backflow prevention device of claim 1, wherein at least any one of the first barrier part (420), the second barrier part (440) and the third barrier part (460) is inclined in the dreg falling direction.

3. The backflow prevention device of claim 1, further comprising:
a cover unit (500) detachably coupled to the reservoir (100), with a handle member (520) rotatably provided on the cover unit (500),
wherein the inflow unit (200) and the discharge unit (300) are formed to pass through a part of the cover unit (500).

4. The backflow prevention device of claim 3, wherein the inflow unit (200) is moved in a direction opposite to the dreg falling direction by rotation of the handle member (520), and blocks a space between the first barrier part (420) and the second barrier part (440).

5. The backflow prevention device of claim 3, wherein, when the handle member (520) is rotated and thus an external force acts in a direction opposite to the dreg falling direction, the inflow unit (200) moves in a direction where the external force acts, thus blocking the space between the first barrier part (420) and the second barrier part (440).

6. The backflow prevention device of claim 1, wherein the discharge unit (300) is coupled to a power unit to suck air from an inside of the reservoir (100).

7. The backflow prevention device of claim 1, wherein the discharge unit (300) comprises a filter member to filter the air.

## Patentansprüche

1. Vorrichtung zum Verhindern eines Rückflusses, die Folgendes umfasst:
einen Behälter (100), der einen Raum definiert, um Rückstände aufzubewahren;
eine Einströmeinheit (200), die ein darin ausgebildetes Eintrittsloch (220) hat und einen Pfad definiert, damit sich die Rückstände durch das Eintrittsloch (220) in den Behälter (100) bewegen können;
eine Abführeinheit (300), die ein darin ausgebildetes Austrittsloch (320) hat und einen Pfad definiert, damit Luft im Behälter (100) durch das Austrittsloch (320) abgeführt werden kann; und
eine Barriereeinheit (400), die im Behälter (100) vorgesehen ist, um eine Strömungsrichtung der Rückstände und/oder der Luft wenigstens einmal zu ändern und dadurch zu verhindern, dass Rückstände in die Abführeinheit (300) strömen,
**dadurch gekennzeichnet, dass** die Barriereeinheit (400) Folgendes umfasst:
ein erstes Barriereteil (420), das von der Außenseite der Einströmeinheit (200) zum Inneren des Behälters (100) ausgebildet ist;
ein zweites Barriereteil (440), das vom Inneren des Behälters (100) zur Außenseite der Einströmeinheit (200) ausgebildet ist; und
ein drittes Barriereteil (460), das von der Außenseite der Einströmeinheit (200) zum Inneren des Behälters (100) ausgebildet ist,
wobei das zweite Barriereteil (440) zwischen dem ersten Barriereteil (420) und dem dritten Barriereteil (460) vorgesehen ist,
wobei die Einströmeinheit (200) so vorgesehen ist, dass ein erstes Ende beweglich ist und sich die Einströmeinheit (200) dann, wenn eine äußere Kraft auf die Einströmeinheit (200) in einer Richtung entgegengesetzt zur Fallrichtung der Rückstände wirkt, in einer Richtung bewegt, in der die äußere Kraft wirkt, wodurch der Raum zwischen dem ersten Barriereteil (420) und dem zweiten Barriereteil (440) blockiert wird.

2. Vorrichtung zum Verhindern eines Rückflusses nach Anspruch 1, wobei das erste Barriereteil (420), das zweite Barriereteil (440) und/oder das dritte Barriereteil (460) in der Fallrichtung der Rückstände geneigt sind.

3. Vorrichtung zum Verhindern eines Rückflusses nach Anspruch 1, die ferner Folgendes umfasst:
eine Abdeckeinheit (500), die mit dem Behälter (100) abnehmbar gekoppelt ist, mit einem Griffelement (520), das an der Abdeckeinheit (500) drehbar vorgesehen ist,
wobei die Einströmeinheit (200) und die Abführeinheit (300) so ausgebildet sind, dass sie durch ein Teil der Abdeckeinheit (500) verlaufen.

4. Vorrichtung zum Verhindern eines Rückflusses nach Anspruch 3, wobei die Einströmeinheit (200) durch eine Drehung des Griffelements (520) in einer Richtung entgegengesetzt zur Fallrichtung der Rückstände bewegt wird und einen Raum zwischen dem ersten Barriereteil (420) und dem zweiten Barriereteil (440) blockiert.

5. Vorrichtung zum Verhindern eines Rückflusses nach Anspruch 3, wobei sich die Einströmeinheit (200) dann, wenn das Griffelement (520) gedreht wird und folglich eine äußere Kraft in einer Richtung entgegengesetzt zur Fallrichtung der Rückstände wirkt, in einer Richtung bewegt, in der die äußere Kraft wirkt, wodurch der Raum zwischen dem ersten Barriereteil (420) und dem zweiten Barriereteil (440) blockiert wird.

6. Vorrichtung zum Verhindern eines Rückflusses nach Anspruch 1, wobei die Abführeinheit (300) mit einem Netzteil gekoppelt ist, um Luft aus dem Inneren des Behälters (100) zu saugen.

7. Vorrichtung zum Verhindern eines Rückflusses nach Anspruch 1, wobei die Abführeinheit (300) ein Filterelement zum Filtern der Luft umfasst.

## Revendications

1. Dispositif de prévention de reflux comportant :
un réservoir (100) définissant un espace pour stocker des résidus dans celui-ci ;
une unité d'écoulement d'entrée (200) ayant un orifice d'entrée (220) formé dans celle-ci, et définissant un trajet pour permettre aux résidus de passer à travers l'orifice d'entrée (220) dans le réservoir (100) ;
une unité d'évacuation (300) ayant un orifice de sortie (320) formé dans celle-ci, et définissant un trajet pour permettre d'évacuer de l'air dans le réservoir (100) à travers l'orifice de sortie (320) ; et
une unité de barrière (400) agencée dans le réservoir (100) pour changer au moins une fois une direction d'écoulement d'au moins un élément parmi les résidus et l'air et empêcher ainsi les résidus de s'écouler dans l'unité d'évacuation (300)
**caractérisé en ce que** l'unité de barrière (400) comporte :
une première partie de barrière (420) formée depuis l'extérieur de l'unité d'écoulement d'entrée (200) vers l'intérieur du réservoir (100) ;
une deuxième partie de barrière (440) formée depuis l'intérieur du réservoir (100) vers l'extérieur de l'unité d'écoulement d'entrée (200) ; et
une troisième partie de barrière (460) formée depuis l'extérieur de l'unité d'écoulement d'entrée (200) vers l'intérieur du réservoir (100),
dans lequel la deuxième partie de barrière (440) est agencée entre la première partie de barrière (420) et la troisième partie de barrière (460),
dans lequel l'unité d'écoulement d'entrée (200) est agencée de telle sorte qu'une première extrémité de celle-ci est mobile et, lorsqu'une force externe agit sur l'unité d'écoulement d'entrée (200) dans une direction opposée à la direction de chute de résidus, l'unité d'écoulement d'entrée (200) se déplace dans une direction où la force externe agit, en bloquant ainsi l'espace entre la première partie de barrière (420) et la deuxième partie de barrière (440).

2. Dispositif de prévention de reflux selon la revendication 1, dans lequel au moins une partie quelconque parmi la première partie de barrière (420), la deuxième partie de barrière (440) et la troisième partie de barrière (460) est inclinée dans la direction de chute de résidus.

3. Dispositif de prévention de reflux selon la revendication 1, comportant en outre :
une unité de couvercle (500) couplée au réservoir (100) de façon détachable, avec un élément de poignée (520) agencé sur l'unité de couvercle (500) de manière rotative,
dans lequel l'unité d'écoulement d'entrée (200) et l'unité d'évacuation (300) sont formées pour passer à travers une partie de l'unité de couvercle (500).

4. Dispositif de prévention de reflux selon la revendication 3, dans lequel l'unité d'écoulement d'entrée (200) est déplacée dans une direction opposée à la direction de chute de résidus par rotation de l'élément de poignée (520), et bloque un espace entre la première partie de barrière (420) et la deuxième partie de barrière (440).

5. Dispositif de prévention de reflux selon la revendication 3, dans lequel, lorsque l'élément de poignée (520) tourne et qu'une force externe agit ainsi dans une direction opposée à la direction de chute de résidus, l'unité d'écoulement d'entrée (200) se déplace dans une direction où la force externe agit, en bloquant ainsi l'espace entre la première partie de barrière (420) et la deuxième partie de barrière (440).

6. Dispositif de prévention de reflux selon la revendication 1, dans lequel l'unité d'évacuation (300) est couplée à un bloc moteur pour aspirer de l'air depuis un intérieur du réservoir (100).

7. Dispositif de prévention de reflux selon la revendication 1, dans lequel l'unité d'évacuation (300) comporte un élément filtrant pour filtrer l'air.
